# FASCICULE DE BREVET EUROPEEN

(11) **EP 3 365 034 B1**
(45) Date de publication et mention de la délivrance du brevet: **04.12.2019**
(21) Numéro de dépôt: 16798240.4
(22) Date de dépôt: 18.10.2016
(51) Int. Cl.: A61L 27/06, A61L 27/50, C09D 4/06, C08F 292/00, C08L 51/10

(54) **PROCÉDÉ POUR GREFFER UN POLYMÈRE BIOACTIF SUR DES IMPLANTS**
VERFAHREN ZUM AUFPFROPFEN EINES BIOAKTIVEN POLYMERS AUF IMPLANTATE
METHOD FOR GRAFTING A BIOACTIVE POLYMER ONTO IMPLANTS

(30) Priorité: 22.10.2015 FR 1560108
(43) Date de publication de la demande: 29.08.2018
(73) Titulaire: Les Laboratoires Osteal Medical, 95957 Roissy Aeroport CDG (FR)
(72) Inventeur: BLANQUAERT, Daniel, 75008 Paris (FR); DE LAMBERT, Bertrand, 60300 Senlis (FR)
(74) Mandataire: CAPRI
(86) Numéro de dépôt international: PCT/FR2016/052682
(87) Numéro de publication internationale: WO 2017/068272

(56) Documents cités:
- WO-A2-2007/141460
- LARY G ET AL: "A bioactive polymer grafted on titanium oxide layer obtained by electrochemical oxidation. Improvement of cell response", JOURNAL OF MATERIALS SCIENCE: MATERIALS IN MEDICINE, KLUWER ACADEMIC PUBLISHERS, BO, vol. 21, no. 2, 20 octobre 2009 (2009-10-20), pages 655-663, XP019770744, ISSN: 1573-4838
- YANG B ET AL: "Preparation of bioactive titanium metal via anodic oxidation treatment", BIOMATERIALS, ELSEVIER SCIENCE PUBLISHERS BV., BARKING, GB, vol. 25, no. 6, 1 mars 2004 (2004-03-01), pages 1003-1010, XP004472562, ISSN: 0142-9612, DOI: 10.1016/S0142-9612(03)00626-4
- DAS ET AL: "Surface modifications and cell-materials interactions with anodized Ti", ACTA BIOMATERIALIA, ELSEVIER, AMSTERDAM, NL, vol. 3, no. 4, 9 juin 2007 (2007-06-09), pages 573-585, XP022110569, ISSN: 1742-7061

## Description

La présente invention concerne un procédé pour greffer un polymère bioactif, le PolyNaSS (polystyrène sulfonate de sodium), sur des implants en titane ou en alliage de titane. Par implants, il faut comprendre toute pièce, ensemble de pièces ou dispositifs destinés à être implantés, partiellement ou en totalité, dans un corps humain ou animal. On peut par exemple citer les implants dentaires, les prothèses de hanche, les prothèses de genou, les prothèses d'épaule, les cages intervertébrales, les pacemakers, etc.

Dans l'art antérieur, on connaît le document EP2032663 qui décrit un procédé de greffage de polymère bioactif sur un matériau prothétique en titane ou alliage de titane. Ce procédé préconise trois étapes successives, à savoir :
- la génération d'espèces actives donneuses de radicaux libres à la surface du matériau prothétique,
- la génération de radicaux à la surface du matériau prothétique par réactions thermiques, et
- la mise en présence du matériau prothétique avec au moins un monomère porteur d'une fonction permettant une polymérisation radicalaire. La polymérisation radicalaire dudit monomère permettant la formation d'un polymère bioactif en l'absence d'oxygène.

Par conséquent, le procédé de ce document EP2032663 est plus particulièrement orienté sur les réactions et interactions physiques ou chimiques permettant de synthétiser le polymère bioactif directement à partir de la surface d'un implant. Cela donne au polymère bioactif la caractéristique d'être greffée de manière permanente sur l'implant. Le polymère bioactif est de préférence du PolyNaSS et le type d'oxydation de l'implant est de préférence une oxydation chimique.

Bien que le procédé du document EP2032663 trace les grandes lignes à suivre pour réaliser le procédé de greffage en laboratoire à petite échelle, il ne donne absolument aucune indication quant à l'application de ce procédé de greffage de manière industrielle à grande échelle.

La présente invention a précisément pour but d'utiliser ce procédé de greffage de polymère bioactif de manière industrielle à grande échelle. En d'autres termes, il s'agit de mettre en œuvre ce procédé de greffage dans un milieu industriel afin de traiter de grandes séries d'implants à la fois. Alors que l'oxydation chimique est mise en avant dans le procédé de greffage du document EP2032663, il s'est rapidement avéré que ce type d'oxydation (chimique) est tout à fait inapproprié, voire dangereux, dans un environnement industriel. La présente invention s'est ainsi tournée vers l'oxydation anodique, qui n'est pas mentionnée dans le document EP2032663.

Afin d'industrialiser le procédé de greffage de polymère bioactif, la présente invention propose les étapes successives suivantes,
a) Monter des implants sur une structure de support d'implants,
b) Plonger les implants dans un bain d'acide pour les décaper,
c) Rincer les implants,
d) Plonger les implants dans un bain d'anodisation pour les anodiser,
e) Rincer les implants,
f) Introduire les implants dans une enceinte de polymérisation remplie de gaz inerte, tel que l'argon,
g) Monter les implants sur un ascenseur présent dans l'enceinte,
h) Actionner l'ascenseur pour plonger les implants dans un bain de polymérisation présent dans l'enceinte,
i) Soumettre le bain de polymérisation à un catalyseur de polymérisation pour synthétiser du polymère bioactif sur les implants,
j) Remonter l'ascenseur pour extraire les implants du bain de polymérisation,
k) Retirer les implants de l'ascenseur,
l) Extraire les implants de l'enceinte,
m) Laver les implants pour les débarrasser de l'excès de polymère bioactif non greffé,
n) Sécher les implants greffés.

Cette succession d'étapes permet une mise en œuvre industrialisée du procédé de greffage qui utilise l'oxydation anodique. La plupart des étapes sont essentielles, voire indispensables pour une mise en œuvre industrialisée, reproductible, efficace, rapide et fiable du procédé de greffage de polymère bioactif.

Des étapes secondaires ou supplémentaires peuvent être mises en œuvre pour améliorer encore davantage le caractère industriel du procédé de greffage.

L'esprit de la présente invention est d'industrialiser un procédé de greffage de polymère bioactif déjà connu du document EP2032663, afin de pouvoir traiter à grande échelle et à grande cadence des implants divers, comme par exemple des implants dentaires ou des implants fémoraux de hanche.

L'invention sera maintenant plus amplement décrite en référence aux dessins joints donnant plusieurs modes de réalisation de l'invention.

Sur les figures :
La figure 1 est une vue très schématique en bloc diagramme représentant les différentes étapes du procédé de greffage selon l'invention,
La figure 2a est une vue en perspective d'un élément de support d'implant selon l'invention,
La figure 2b est une vue de l'élément de support d'implants de la figure 2a avec des implants montés dessus,
La figure 2c est une vue en coupe transversale verticale agrandie à travers un implant monté sur une broche de l'élément de support d'implants des figures 2a et 2b,
La figure 3 est une vue en perspective d'une platine de montage d'éléments de support d'implants qui a déjà reçu trois éléments de supports d'implants et qui est prêt à en recevoir un quatrième,
La figure 4 est une vue en perspective d'un récipient rempli de gaz inerte dans lequel est déposée la platine de montage avec ses éléments de support d'implants,
La figure 5a est une vue en perspective schématique d'une enceinte mise en œuvre pour l'étape de polymérisation,
La figure 5b est une vue en perspective agrandie d'un détail de la figure 5a,
La figure 6 est une vue en perspective d'une plaque de montage dans laquelle les éléments de support d'implants sont reçus par coulissement,
La figure 7 est une vue en plan schématique de la station de polymérisation disposée à l'intérieur de l'enceinte de polymérisation,
Les figures 8a à 8e représentent différents modes de réalisation d'une cuve de polymérisation contenant le monomère,
Les figures 9a et 9b sont des vues en perspective d'un autre mode de réalisation de la station de polymérisation,
Les figures 10a et 10b représentent deux variantes de cuves recevant le dispositif des figures 9a et 9b,
La figure 11 est une vue en perspective d'un panier de lavage selon l'invention,
La figure 12a représente un étrier pour l'anodisation de deux implants fémoraux de hanche,
La figure 12b représente un implant fémoral de hanche disposé dans un récipient rempli de gaz inerte,
La figure 12c représente une réglette sur laquelle sont montés les implants fémoraux de hanche,
La figure 12d représente deux réglettes engagées dans des rails de montage de l'ascenseur de la station de polymérisation, et
La figure 12e représente un casier de lavage pour des implants fémoraux de hanche.

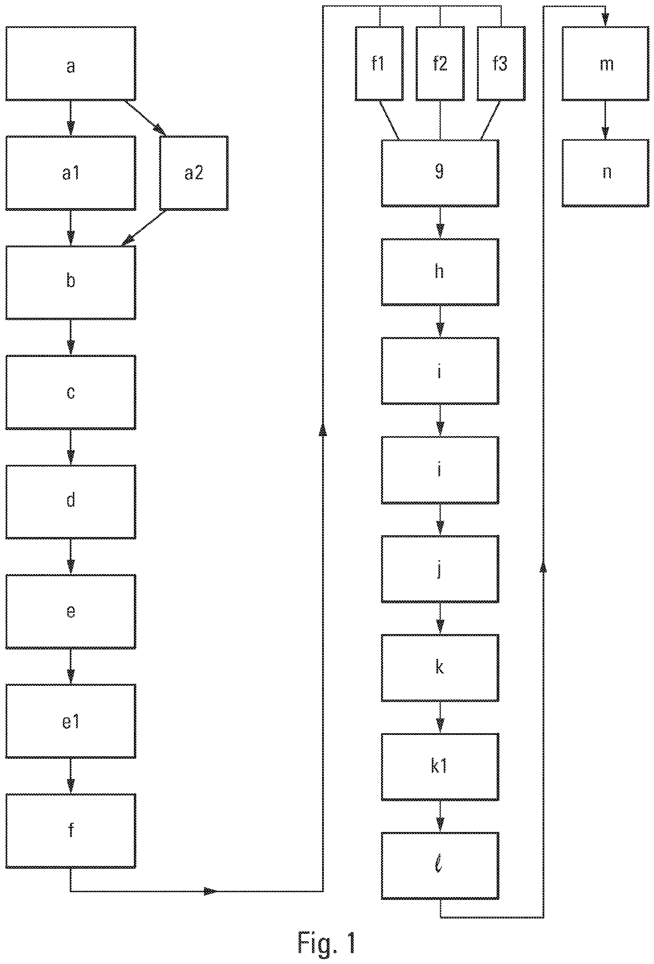

On se référera tout d'abord à la figure 1 pour décrire les différentes étapes successives du procédé de greffage du polymère bioactif, le PolyNaSS (polystyrène sulfonate de sodium), sur des implants en titane ou en alliage de titane, afin de réaliser un revêtement antiadhésif ou « anti-accroche » sur lequel les bactéries et autres agents infectieux glissent de sorte qu'ils ne peuvent pas s'y développer.

Les étapes majeures successives sont les suivantes :
a) Monter des implants sur une structure de support d'implants : on verra ci-après un élément de support pour implants dentaires et un étrier de support pour implants fémoraux de hanche.
b) Plonger les implants (montés sur leur support) dans un bain d'acide, tel que de l'acide nitrique et/ou fluorhydrique, pour les décaper : le temps de trempage peut être de l'ordre de 30 s à 1 minute.
c) Rincer les implants, par exemple à l'eau,
d) Plonger les implants (montés sur leur support) dans un bain d'anodisation, par exemple à base d'acide orthophosphorique, pour les anodiser et ainsi créer des peroxydes de titane en surface: le temps de trempage peut être de l'ordre de 10s à 1 minute,
e) Rincer les implants, par exemple à l'eau,
f) Introduire les implants (encore montés sur leur support, ou un autre support, ou sur aucun support) dans une enceinte de polymérisation étanche remplie de gaz inerte, tel que l'argon : un autre gaz inerte peut aussi être utilisé.
g) Monter les implants (montés sur leur support, ou un autre support, ou sur aucun support) sur un ascenseur installé dans l'enceinte étanche,
h) Actionner l'ascenseur pour plonger les implants dans un bain de polymérisation, par exemple de monomère, le styrène sulfonate de sodium (NaSS), présent dans l'enceinte,
i) Soumettre le bain de polymérisation à un catalyseur de polymérisation, par exemple thermique ou UV, pour synthétiser du polymère bioactif à la surface des implants, et ainsi obtenir un implant revêtu d'une couche de polymère greffé, PolyNaSS,
j) Remonter l'ascenseur pour extraire les implants revêtu du bain de polymérisation,
k) Retirer les implants de l'ascenseur,
l) Extraire les implants de l'enceinte étanche,
m) Laver les implants, par exemple par aspersion d'eau pure, pour les débarrasser de l'excès de polymère bioactif non greffé,
n) Sécher les implants greffés.

Outre ces étapes majeures, le procédé définit également des étapes intermédiaires, secondaires et/ou optionnelles qui améliorent encore davantage les étapes majeures ou permettent une manipulation plus aisée d'implants particuliers, comme par exemple des implants dentaires ou encore des implants fémoraux de hanche. On peut notamment citer les étapes suivantes.

Pour les implants dentaires :
- une étape intermédiaire a1- entre l'étape a- et l'étape b-, consistant à monter plusieurs implants sur des éléments de support, eux-mêmes montés sur une platine de support, comprenant avantageusement un manche de préhension amovible, la platine avec ses éléments de support d'implants constituant une structure de support d'implants,
- les implants, lors des étapes b- à f-, sont manipulés au moyen de la platine de support, avec les éléments de support d'implants montés dessus,
- une étape intermédiaire e1- entre l'étape e- et l'étape f-, consistant à placer la platine de support avec ses éléments de support d'implants dans un récipient rempli de gaz inerte, tel que l'argon, avantageusement pourvu d'un couvercle étanche, le récipient étant ensuite introduit lors de l'étape f- dans l'enceinte étanche remplie de gaz inerte, tel que l'argon, le récipient étant alors ouvert pour en extraire la platine de support avec ses éléments de support d'implants,
- une étape intermédiaire f1- entre l'étape f- et l'étape g-, consistant à retirer les éléments de support d'implants de la platine de support, puis à monter les éléments de support d'implants sur une plaque de support qui est ensuite montée sur l'ascenseur, ou, e, variante, une étape intermédiaire f2-entre l'étape f- et l'étape g-, cette étape f2- consistant à monter sur des tiges axiales verticales (C) de l'ascenseur (L') qui sont entraînées en rotation dans le bain de polymérisation, soit la platine de support (B), soit les éléments de support (S).
- une étape intermédiaire k1- entre l'étape k- et l'étape l-, consistant à retirer les éléments de support d'implants de l'ascenseur, puis à monter les éléments de support d'implants sur une poutre centrale formant des logements de montage pour les éléments de support d'implants afin de former un panier le lavage, qui est ensuite extrait de l'enceinte. Le panier de lavage peut également être constitué à la sortie de l'enceinte étanche.
- lors des étapes m- et n-, les éléments de support d'implants sont configurés sous la forme du panier de lavage.

Pour les implants fémoraux de hanche :
- une étape intermédiaire a2- entre l'étape a- et l'étape b-, consistant à monter plusieurs implants (H) sur un étrier de support (Th), les implants (H), lors des étapes b- à f-, étant manipulés au moyen de l'étrier de support (B),
- une étape intermédiaire f3- entre l'étape f- et l'étape g-, consistant à monter les implants côte à côte sur une réglette qui est ensuite montée sur l'ascenseur,
- lors de l'étape l-, les implants sont sur la réglette,
- lors des étapes m- et n-, les implants sont disposés sur un casier de lavage.

A titre d'exemple, ce procédé de greffage de polymère actif sera maintenant mis en œuvre sur des implants dentaires pour lesquels il a été développé des outils, accessoires ou ancillaires permettant une manipulation plus aisée, plus rapide et à grande échelle.

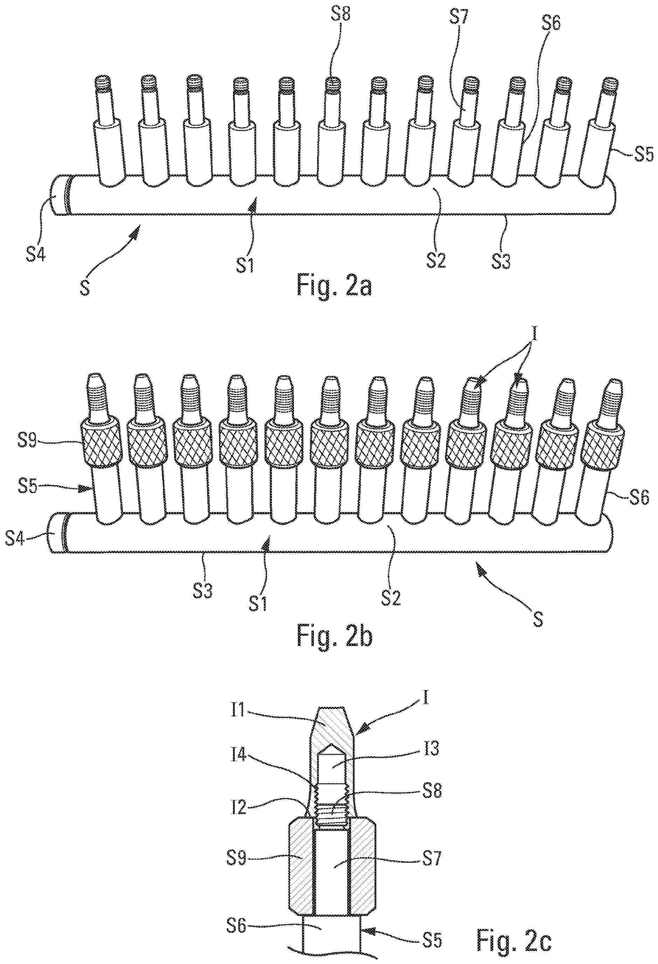

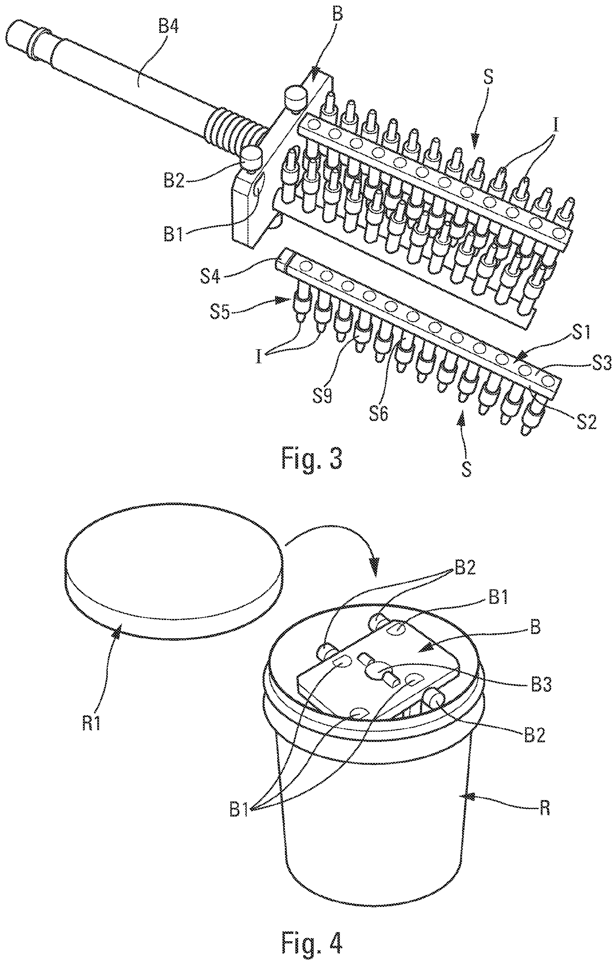

En se référant aux figures 2a, 2b et 2c, on peut voir un élément de support S pour des implants, tels que des implants dentaires I. Un type d'implants I est plus particulièrement visible en coupe sur la figure 2c. On peut voir que l'implant I comprend une tête I1 à son extrémité supérieure et un bord inférieur annulaire I2 à son extrémité opposée. L'implant I définit un intérieur creux I3 présentant une paroi filetée I4. Il s'agit là d'une conception tout à fait classique pour un implant dentaire. L'élément de support S comprend une barrette S1 sur laquelle sont montées plusieurs broches S5 qui s'étendent avantageusement parallèlement les unes aux autres. On peut par exemple prévoir douze broches S5 disposées de manière alignée et parallèle sur une barrette S1 avec un écart entre l'axe de broche de l'ordre de 1 à 2 cm. Selon un mode de réalisation avantageux, la barrette présente une section transversale cylindrique circulaire tronquée à sa base de manière à former un méplat longitudinal qui est disposé de manière opposée aux broches S5. Ceci est plus visible sur la figure 3. La barrette S1 comprend au moins une extrémité de montage pour le montage de la barrette sur une structure de support, qui sera définie ci-après. Cette extrémité de montage S4 peut même présenter un logement creux. Quant aux broches S5, elles comprennent chacune une embase S6 raccordée à la barrette S1, une tigelle S7 montée sur l'embase S5 en définissant un diamètre inférieur et comprenant une extrémité libre pourvue d'un filetage S8. Un épaulement est ainsi formé au niveau de l'extrémité supérieure de l'embase autour de la tigelle S7. Chaque broche S5 est en outre pourvue d'une bague rotative S9 qui est engagée autour de la tigelle S7 en prenant appui sur l'embase S5. L'extrémité libre filetée S8 fait saillie hors de la bague rotative S9 afin de permettre un vissage de l'implant I sur l'extrémité libre filetée en venant en butée sur la bague rotative S9, comme cela est représenté sur la figure 2c. On peut clairement voir que l'extrémité libre filetée S8 est en prise avec le filetage interne I4 du logement creux I3 de l'implant I. Il suffit que l'implant I soit à peine vissé sur l'extrémité libre filetée S8 pour garantir son maintien sur la tigelle S7 en appui sur la bague rotative S9. Les éléments de support S avec les implants I montés dessus, comme représentés sur la figure 2b, présentent une configuration globale de peigne avec des dents constituées par des broches S5 sur lesquelles sont montés les implants I.

Les bagues rotatives S9 constituent selon l'invention un système de retrait pour retirer les implants des moyens de réception de l'élément de support constitué par les extrémités libres filetées S8. En effet, en entraînant les bagues rotatives S9 en rotation libre autour des tigelles S7, il s'ensuit un desserrage et un dévissage des implants I des extrémités supérieures filetées S8, et ce, sans qu'il soit nécessaire de venir en contact avec les implants I, et notamment avec leur partie exposée. En d'autres termes, les bagues rotatives permettent le dévissage des implants sans avoir à les toucher. Il est avantageux que les bagues rotatives S9 soient parfaitement alignées de sorte qu'une action commune et simultanée est possible sur l'ensemble des bagues rotatives pour dévisser simultanément tous les implants I d'un élément de support d'implants S. On peut par exemple imaginer une baguette rectiligne qui est mise en contact appuyé de l'ensemble des bagues rotatives S9 et à laquelle on imprime un mouvement rapide de va-et-vient, permettant d'assurer le dévissage des implants I. Les implants I peuvent alors tomber par gravité dans un récipient de récupération. Il va de soi que cette opération de retrait des implants I ne se fera qu'à la fin du procédé de greffage, après l'étape n de séchage.

Sans sortir du cadre de l'invention, on peut également imaginer que les implants I soient dépourvus de filetage interne I4 et que la barrette S1 et les broches S5 soient creuses de manière à pouvoir générer une dépression qui va plaquer les implants I sur les extrémités libres des tigelles S7. La dépression peut par exemple être créée au niveau de l'extrémité de montage S4. Dans ce cas, il n'est pas nécessaire de prévoir de système de retrait, tel que les bagues rotatives S9. Toutefois, l'application d'une dépression nécessite une mise en œuvre plus compliquée, c'est pourquoi l'utilisation de bagues rotatives S9 est une mise en œuvre plus simple et efficace.

En se référant à la figure 3, on peut voir trois éléments de support S montés sur une platine de montage B comprenant plusieurs logements de montage B1 dans lesquels les extrémités de montage S4 des éléments de support S sont engagées et avantageusement bloquées aux moyens de vis de blocage B2. La figure 3 représente également un quatrième élément de support S prêt à être engagé dans son logement de montage B1 encore libre. Ainsi, quatre éléments de support S peuvent être montés sur une platine de montage avec une disposition mutuelle tête bêche. En effet, les deux éléments de support S les plus hauts sont disposés avec leurs broches S5 dirigées vers le bas, alors que les deux autres éléments de support S montés en bas sont disposés avec leurs broches S5 orientées vers le haut. Selon l'invention, la platine de montage B est pourvue d'un manche de préhension amovible B4 qui permet une manipulation aisée de la platine de montage B avec ses éléments de support S montés dessus.

On peut notamment utiliser la platine de montage B avec son manche amovible B4 lors des étapes a- à e- du procédé de greffage de polymère bioactif. On peut même se servir de la platine de montage B avec son manche amovible B4 pour disposer la platine de montage B avec ses éléments de support S dans le récipient rempli de gaz inerte tel que de l'argon. Ceci est visible sur la figure 4. Le manche amovible B4 doit alors être retiré laissant apparaître son logement de montage B3 au niveau de la platine B. Le récipient R peut être obturé à l'aide d'un couvercle R1 : ceci est toutefois optionnel, car l'argon est plus lourd que l'air et reste dans le récipient R même en l'absence de couvercle. Ainsi, la platine de montage B avec ses quatre éléments de support S sont introduits à l'intérieur de l'enceinte de polymérisation E qui est remplie de gaz inerte, tel que de l'argon, en étant disposé dans le récipient R. A l'intérieur de l'enceinte E, le couvercle R1 peut être retiré et la platine de montage B avec ses éléments de support S peut être extraite du récipient R.

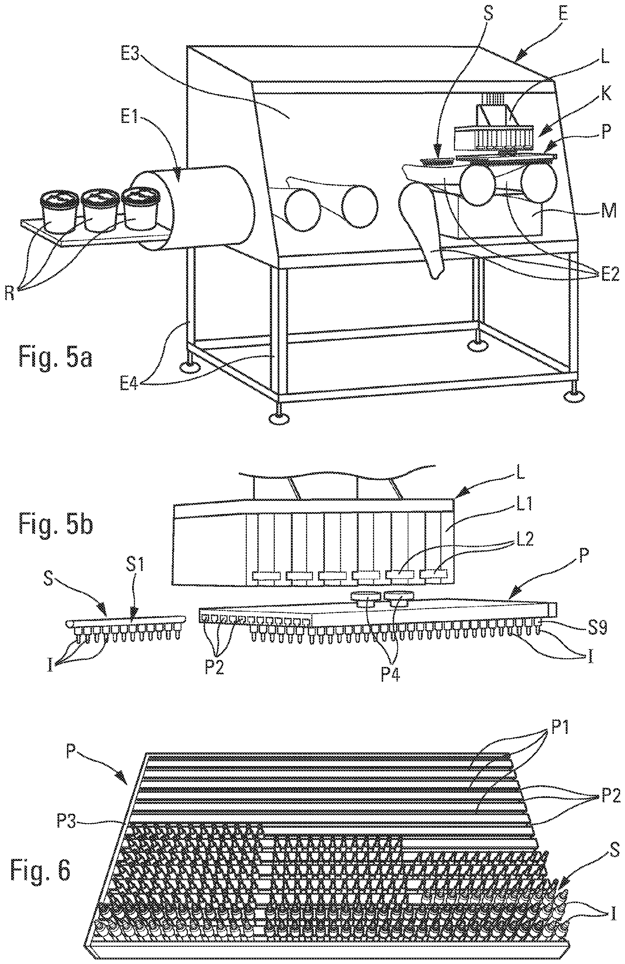

L'enceinte de polymérisation E est visible sur la figure 5a. Ce type d'enceinte est communément désigné sous l'expression « boite à gants », en raison de la présence de gants qui permettent d'effectuer des manipulations à l'intérieur de l'enceinte E à travers une paroi vitrée E3. Bien que non représentée, l'enceinte E est pourvue de moyens de traitement de son atmosphère interne afin de garantir des conditions optimales de pression, de pureté, et/ou d'hygrométrie. Le gaz le plus couramment utilisé est l'argon, bien que d'autres gaz peuvent également être mis en œuvre. L'enceinte E est montée sur un piétement E4 et comprend un sas d'entrée E1 à travers lesquels les récipients R passent pour parvenir à l'intérieur de l'enceinte E. Le sas E1 comprend de manière conventionnelle une porte d'entrée et une porte de sortie afin de pouvoir contrôler l'atmosphère qui règne à l'intérieur du sas E1.

L'enceinte de polymérisation E contient une station de polymérisation K au niveau de laquelle les implants I sont plongés dans un bain de polymérisation pour permettre la synthèse de polymères bioactifs (par exemple du PolyNaSS) à la surface anodisée des implants, à partir d'un monomère X approprié, tel que du styrène sulfonate de sodium (NaSS). Cette station de polymérisation K comprend un ascenseur L qui est déplaçable verticalement au-dessus d'une cuve T qui est remplie de monomère X pour plonger/extraire les implants I dans et hors du bain de polymérisation de la cuve T. Cette station de polymérisation K comprend avantageusement des moyens catalyseurs pour accélérer la polymérisation sur les implants plongés dans le bain. Ces moyens catalyseurs peuvent se présenter sous la forme d'un bac de bain-marie M rempli de liquide O qui est chauffé par des moyens de chauffage M1, comme visible sur la figure 6. Les moyens catalyseurs peuvent également prendre la forme d'une source de rayonnement UV, comme on le verra ci-après.

En se référant simultanément aux figures 5b, 6 et 7 , on peut voir que l'ascenseur L comprend un chariot mobile verticalement L1 qui est monté sur une crémaillère verticale L4 de manière à permettre le déplacement vertical de haut en bas et de bas en haut du chariot L1. Ce chariot mobile est pourvu de moyens de montage aptes à recevoir une structure de support d'implants, comme on va le voir ci-après. Ces moyens de montage peuvent par exemple comprendre plusieurs glissières L2 dans lesquelles des moyens de fixation P4 de la structure de support sont insérables par coulissement. Les glissières L2 forment des ouvertures d'accès sur une face frontale tournée vers les gants de manipulation E2. L'ascenseur L peut optionnellement être pourvu d'un couvercle L3 qui vient coiffer, avantageusement de manière étanche, à la fois la cuve T remplie de monomère X et le bac de bain-marie M rempli de liquide chauffé O.

Dans le cas où le procédé de greffage de l'invention est appliqué à des implants dentaires, et plus particulièrement à des éléments de support S tels que décrits précédemment, il est prévu une plaque de montage P comprenant, sur une de ses faces, plusieurs rails de montage P1 dans lesquels les barrettes S1 des éléments de support S sont engagés par coulissement à partir d'une extrémité ouverte d'accès P2, comme cela est visible sur la figure 6 . Les éléments de support S peuvent ainsi être engagés les uns derrière les autres et les uns à côté des autres dans les rails de montage P1 de la plaque de montage P jusqu'à ce qu'elle soit pleine. Les broches S5 avec leurs implants montés dessus font saillie hors des rails de montage P1. La plaque de montage P est alors renversée et elle peut être montée dans les glissières L2 du chariot L1 au moyen d'un ou de plusieurs talon(s) de fixation P4. De cette manière, la plaque de montage P est fixée et disposée en dessous du chariot L1. L'ascenseur L peut alors être abaissé de manière à plonger les implants I dans le bain de monomères X. Le temps de polymérisation varie de 2 à 15 heures en fonction des moyens catalyseurs utilisés.

Selon un détail de manipulation, les éléments de support S sont retirés de la platine de support B à l'intérieur de l'enceinte E pour être montés individuellement dans les rails de montage P1 de la plaque de support P.

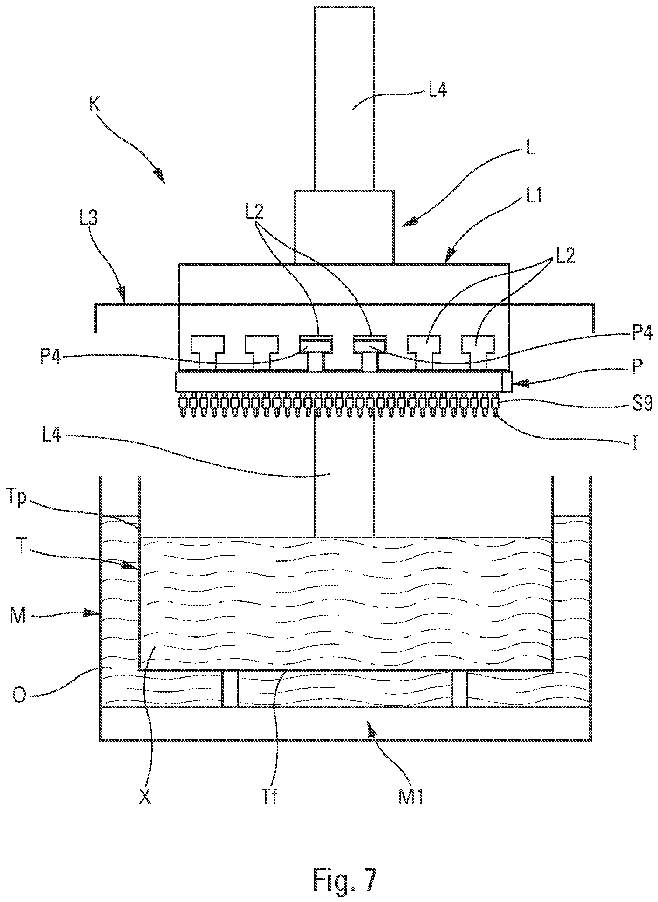

La cuve T de la figure 7, qui contient le monomère X, peut par exemple présenter une forme parallélépipédique avec un fond plat Tf et quatre parois latérales Tp. Avec des moyens catalyseurs sous la forme d'un bac de bain marie M, cette forme de réalisation est adéquate. Afin d'uniformiser la température à l'intérieur du bac de la cuve T, on peut prévoir des moyens générateurs de courant, comme par exemple un agitateur.

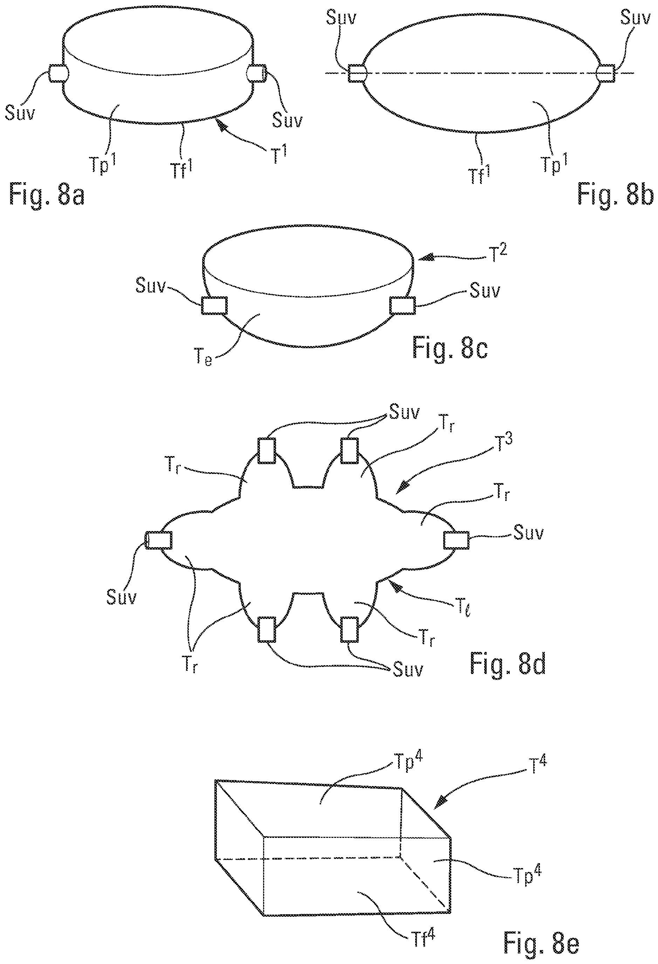

Les figures 8a à 8e illustrent de manière très schématique plusieurs variantes de réalisation pour une cuve remplie de monomère X dont les moyens catalyseurs se présentent sous la forme de sources de rayonnement UV. Sur les figures 8a et 8b, la cuve T¹ présente un fond plat Tf¹, mais une paroi latérale Tp¹ de forme elliptique. Deux sources de rayonnement UV Suv sont disposées l'une en face de l'autre sur le grand axe de l'ellipse, comme représenté sur la figure 8b. La forme elliptique de la paroi latérale Tp¹ permet d'améliorer la propagation des rayonnements UV à travers le monomère, particulièrement lorsque la paroi latérale Tp¹, et éventuellement aussi le fond Tf¹, sont réfléchissants. On peut par exemple prévoir une cuve T¹ en inox avec un fini miroir. On peut également prévoir un revêtement réfléchissement argenté à l'intérieur de la cuve T¹.

La figure 8c représente une variante dans laquelle la cuve T² présente une paroi Te en forme de coupelle ou d'auge, sans fond plat. Elle peut par exemple correspondre à une demie ellipsoïde de révolution. Les sources de rayonnement UV S_{UV} sont également disposées l'une en face de l'autre sur le grand axe de l'ellipsoïde, comme dans le mode de réalisation des figures 8a et 8b.

La figure 8d représente encore une autre variante de réalisation dans laquelle la cuve T³ est formée de plusieurs réflecteurs elliptiques Tr raccordés les uns aux autres par des tronçons de liaison Tl. La paroi latérale de la cuve T³ présente ainsi une forme complexe. Chaque réflecteur Tr est pourvu d'une source de rayonnement UV S_{UV}. La paroi latérale peut être réfléchissante, avec un fini miroir ou un revêtement argenté.

Sur la figure 8e représente une autre variante dans laquelle la cuve T⁴ est de forme parallélépipédique avec un fond plat Tf⁴ et les parois latérales Tp⁴. Une ou plusieurs de ces parois peuvent être constituées d'un panneau UV. On peut même imaginer que toute la cuve T⁴ soit constituée de cinq panneaux UV raccordés les uns autres aux autres.

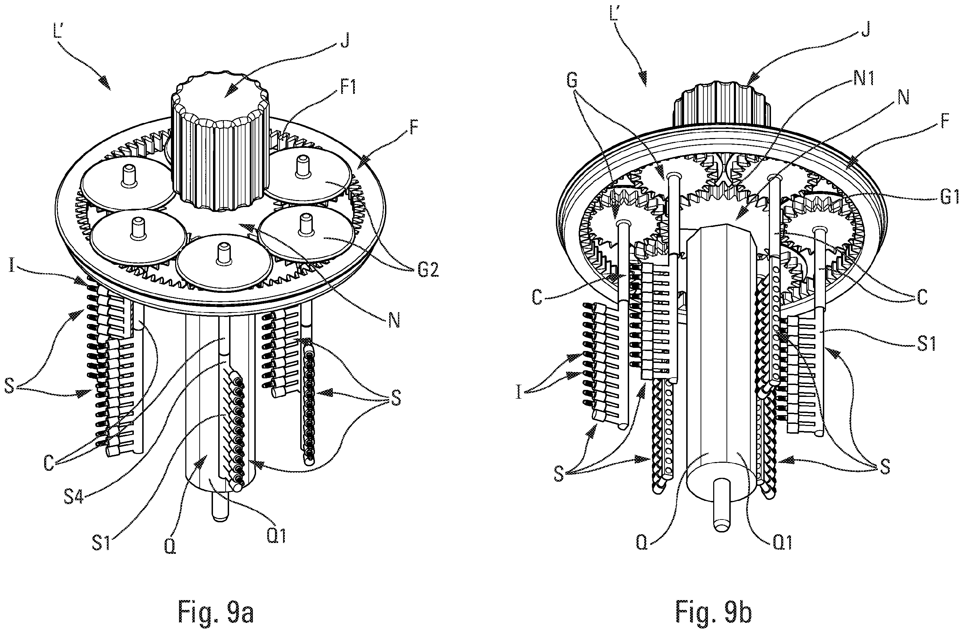

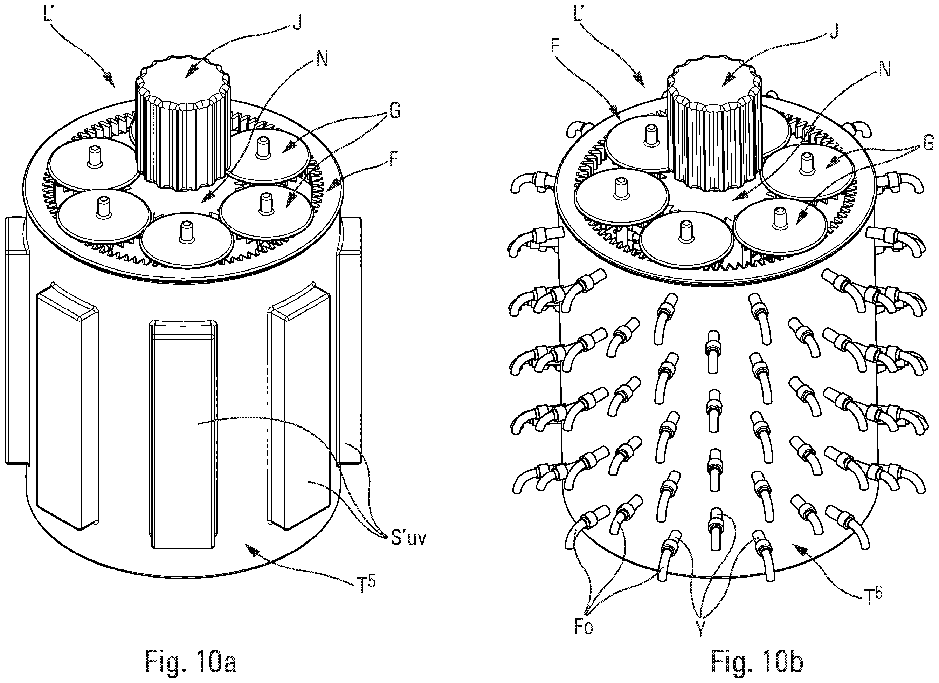

On se référera aux figures 9a, 9b, 10a et 10b pour décrire un second mode de réalisation pour un chariot L1' utilisable dans le cadre de la présente invention. Ce chariot L1' peut être mis en œuvre à la place du chariot L1 précédemment décrit, qui est disposé à l'intérieur de l'enceinte étanche E. Alors que les implants I sont statiques dans la cuve lorsque le chariot L1 est dans sa position basse, les implants I montés sur le chariot L1 ' sont déplaçables à l'intérieur de la cuve, lorsque le chariot L1' est en position basse. En se référant aux figures 10a et 10b, on peut voir que les deux versions de cuves T⁵ et T⁶ présentent une forme sensiblement cylindrique avec des sources de rayonnement UV réparties sur sa périphérie. La cuve T⁵ comprend ainsi huit sources UV S'_{UV} qui sont réparties de manière équidistante tout autour de la cuve. Les sources S'_{UV} peuvent présenter une étendue axiale importante. Quant à la cuve T⁶, elle est pourvue d'une pluralité d'embouts optiques Y auxquels sont raccordées des fibres optiques Fo reliées à une source UV (non représentée). Compte tenu du nombre important d'embouts Y et de fibres optiques Fo, l'intérieur de la cuve T⁶ est irradiée de manière parfaitement homogène. La paroi interne de ces cuves T⁵ et T⁶ peuvent être réfléchissantes pour le rayonnement UV.

Sur les figures 10a et 10b, le chariot L1' est représenté dans sa position basse, dans lequel il est introduit au maximum à l'intérieur des cuves T⁵ et T⁶. Sur ces figures, il n'est pas représenté de quelle manière le chariot L1' est déplacé axialement verticalement, mais n'importe quel moyen approprié peut être utilisé.

On se référera maintenant aux figures 9a et 9b pour décrire en détail la structure et le fonctionnement de ce chariot L1'. Il est ici destiné à recevoir des éléments de support S tels que décrits précédemment, mais il peut également être envisageable que des platines de support B dotées de plusieurs éléments de support S soient reçus par le chariot L1'. Dans le cas particulier des figures 9a et 9b, chaque élément de support S est monté par son extrémité de montage S4 à l'extrémité libre d'une tige axiale verticale C qui forme l'axe de rotation d'une roue horizontale G. Chaque roue G est dotée d'un disque supérieur G1 à travers lequel débouche l'extrémité supérieure de la tige axiale verticale C. Sur les figures 9a et 9b, les roues horizontales G sont en nombre de six, mais ce nombre n'est pas limitatif. Ces roues horizontales G sont disposées à l'intérieur d'une couronne de révolution F qui est dentée intérieurement en F1. Les dents G1 des roues horizontales G sont en prise avec les dents F1 de la couronne de révolution F. Le disque G2 repose avec leur bord externe sur la couronne de révolution F. D'autre part, les roues dentées G sont également en prise avec une roue d'entraînement centrale N qui est également dentée en N1. Cette roue centrale N est pourvue en partie haute d'un plot d'entraînement J destiné à venir en prise avec des moyens d'entraînement en rotation (non représentés). A son extrémité inférieure, la roue d'entraînement N est pourvue d'un moyeu Q qui s'étend entre les tiges axiales verticales C et les éléments de support S. Avantageusement, le moyeu Q est réfléchissant, tout particulièrement aux rayonnements UV. On peut par exemple réaliser le moyeu Q avec des facettes réfléchissantes Q1. Bien que non représenté, le chariot L1' peut être pourvu d'un couvercle qui coiffe hermétiquement les roues horizontales G et la couronne de révolution F.

Avec une telle conception, on comprend aisément que l'entraînement en rotation du plot d'entraînement J a pour effet d'entraîner la roue d'entraînement N en rotation sur elle-même. Etant donné que la roue d'entraînement N est en prise engrenée avec les roues horizontales G, ces dernières sont entraînées en rotation à la fois sur elle-même, mais également autour de la roue d'entraînement N à l'intérieur de la couronne de révolution F. Par conséquent, les éléments de support S sont entraînés à la fois en rotation autour d'un axe passant par les tiges C et leurs barrettes S1, mais également en rotation de révolution autour d'un axe central passant par le plot d'entraînement J, la roue d'entraînement N et le moyeu Q. Les éléments de support S effectuent donc un mouvement complexe résultant de la combinaison d'une rotation autour de leurs barrettes et d'une révolution autour de la roue d'entraînement centrale N. De cette manière, les implants I montés sur les éléments de support S se déplacent selon une trajectoire complexe à l'intérieur du monomère présent dans la cuve. On garantit ainsi que les implants I sont exposés de manière identique et homogène au rayonnement UV qui irradie le monomère présent dans la cuve.

Avec un ascenseur L' associé à une cuve irradiée par un rayonnement UV, comme les cuves T⁵ et T⁶, on obtient un revêtement greffé de polymère bioactif, tel que du PolyNaSS, avec une épaisseur et une densité souhaitées. Le temps nécessaire pour une polymérisation satisfaisante peut être considérablement réduit par rapport à une polymérisation avec catalyseur thermique (du type bain-marie) qui est de l'ordre de 15 heures. On peut en effet réduire aisément ce temps de moitié, et même davantage avec un rayonnement UV et une cuve optimisés, pour atteindre un temps de l'ordre de 2 à 5 heures, voire encore moins de l'ordre de 1 heure.

Une fois le greffage achevé, les implants sont extraits de la cuve en remontant le chariot L1 ou L1'. Les éléments de support S peuvent alors être retirés du chariot en les manipulant à l'aide des gants de manipulation E2. Ils peuvent être ainsi amenés dans le sas d'entrée E1, d'où ils sont extraits de l'enceinte étanche E. Ils peuvent ensuite subir les étapes de lavage pour les débarrasser de l'excès de polymère bioactif greffé et de séchage.

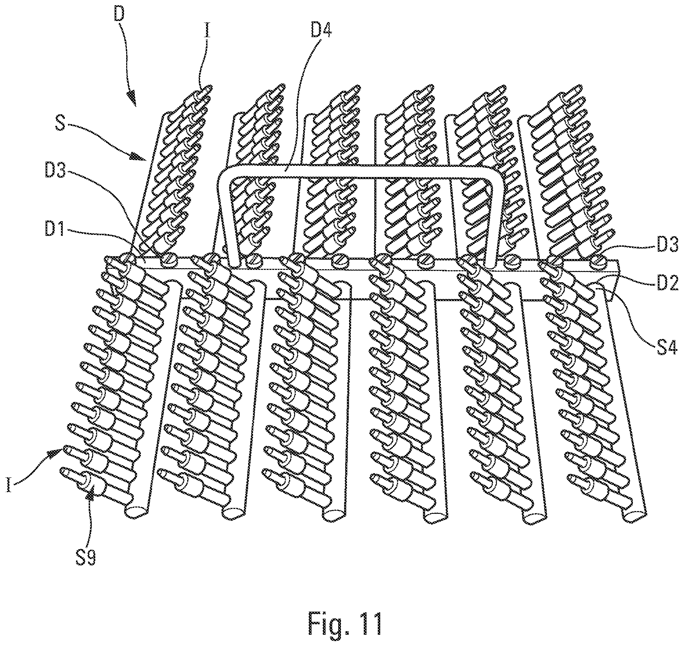

En se référant à la figure 11, on peut voir un autre ustensile ou ancillaire qui permet une manipulation avantageuse des éléments de support S lors des étapes de lavage et de séchage. Cet ustensile peut être qualifié de panier de lavage D, étant donné qu'il présente une configuration proche de celle des paniers que l'on rencontre dans les lave-vaisselles. Ce panier de lavage D comprend une poutre centrale D1 qui forme plusieurs logements de montage D2 dans lesquels sont engagées les extrémités de montage S4 des éléments de support S. Des vis de blocage optionnels D3 peuvent être prévus pour bloquer les extrémités de montage S4 à l'intérieur des logements de montage D2. La poutre centrale D1 peut également être pourvue d'une anse de préhension D4 par laquelle on peut saisir manuellement le panier de lavage D. Sur la figure 11, on peut dénombrer douze éléments de support S, à savoir six de chaque côté de la poutre centrale D1. On peut remarquer que les éléments de support S sont disposés avec une orientation inclinée, de l'ordre de 45° par rapport à l'horizontale ou la verticale. L'inclinaison des éléments de support S de part et d'autre de la poutre centrale D1 sont opposés, ou décalés de 90°. En effet, les implants I visibles en dessous de la poutre centrale D1 sur la figure 11 sont orientés vers la gauche, alors que les implants I disposés au-dessus de la poutre centrale D1 sont orientés vers la droite.

Ce panier de lavage D peut ainsi aisément être disposé dans un équipement de lavage approprié, dans lequel de l'eau, de préférence hautement purifiée, est projetée par aspersion sur les implants I, afin de les débarrasser de leur excès de polymère bioactif greffé. Cette phase de lavage est suivie par une phase de séchage qui peut être opérée dans le même appareil de lavage.

A la fin de l'étape de séchage, le panier D est extrait de l'appareil de lavage/séchage, et les éléments de support S sont retirés de la poutre centrale D1. Ensuite, les implants I peuvent être retirés de l'élément de support S, comme précédemment expliqué, en entraînant les bagues rotatives S9 en rotation, afin de dévisser les implants I des extrémités filetées S8. Sans sortir du cadre de l'invention, on peut également imaginer que les implants I soient maintenus par aspiration sur des éléments de support appropriés.

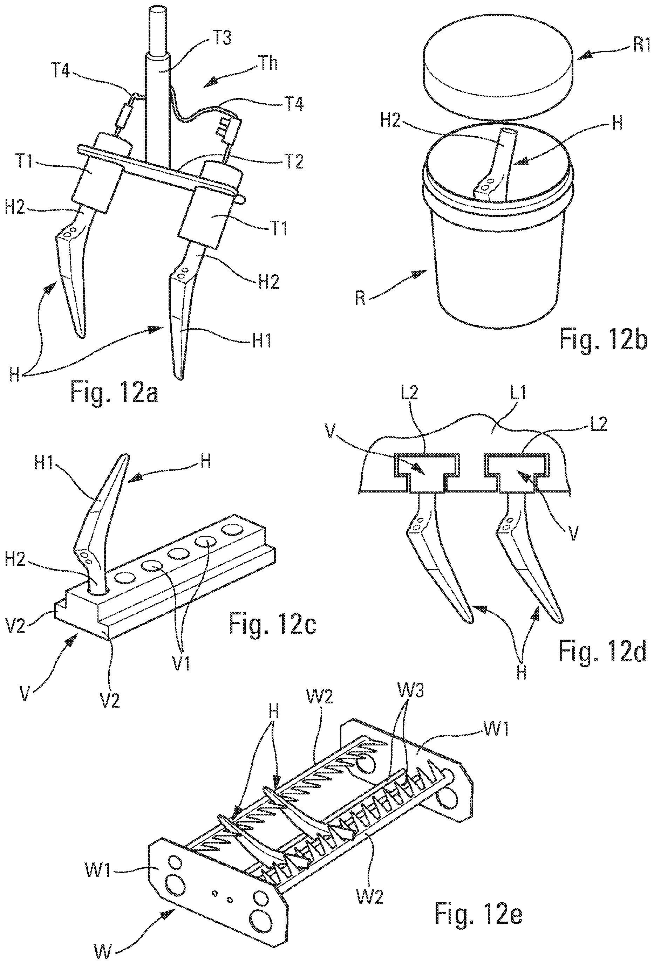

Jusqu'à présent, toute la description a été faite en référence à un type d'implant particulier, à savoir les implants dentaires I. L'invention n'est toutefois pas limitée à ce type d'implant particulier, et d'autres types d'implants peuvent être traités, revêtus et manipulés selon l'invention. En se référant aux figures 12a à 12f, il est fait référence à un autre type d'implant, à savoir un implant fémorale de hanche H. On peut également parler de prothèse fémorale. Cet insert H comprend de manière typique une broche fémorale H1 destinée à être insérée et scellée dans le fémur et une tige de col H2 destinée à recevoir la tête fémorale qui est reçue dans la cotyle fixé au bassin. Les prothèses fémorales H ici traitées sont dépourvues de têtes.

En se référant à la figure 12a, on peut voir un premier dispositif de support sous la forme d'un étrier Th qui permet de recevoir deux implants fémoraux H. Plus précisément, cet étrier Th comprend deux manchons de réception T1 dans lesquels sont reçues respectivement les tiges de col H2 des implants H. Ces manchons T1 sont montés sur une plaquette commune qui est pourvue d'une tige de montage T3. Les deux manchons T1 sont également pourvus de moyens de câblage électriques T4 pour alimenter les inserts H en courant. Cet étrier Th peut être utilisé lors des étapes b, c, d, et e du procédé de greffage. Cet étrier Th constitue une structure de support d'implants, au même titre que l'élément de support S, la platine de support B et la plaque de support précédemment décrits. L'étrier Th peut être monté sur un ascenseur au moyen de sa barre de montage T3 pour réaliser les étapes de décapage, d'anodisation et de rinçage.

Une fois ces étapes terminées, les implants H peuvent être retirés de l'étrier Th et placés individuellement ou en groupe dans un récipient R, tel que celui utilisé précédemment pour les implants dentaires. Ce récipient R est avantageusement rempli d'un gaz inerte, tel que de l'argon, et pourvu optionnellement d'un couvercle R1. Les implants peuvent ainsi être introduits dans l'enceinte de polymérisation E à travers le sas d'entrée E1. A l'intérieur de l'enceinte, les implants H peuvent être montés sur une réglette V, visible sur la figure 12c. Cette réglette V comprend des trous de réception V1 dans lesquels les tiges de col H2 sont insérées en force. La réglette V comprend également deux brides longitudinales opposées V2, qui vont permettre l'insertion par coulissement des réglettes V dans les glissières L2 du chariot L1 de l'ascenseur L, comme visible sur la figure 12d. Les implants H peuvent ainsi être plongés dans la cuve remplie de monomère X. Après un temps de polymérisation suffisant, les implants peuvent être extraits de la cuve et les réglettes V peuvent être extraites des glissières L2. Les implants H peuvent ensuite être retirés des réglettes V et extraits de l'enceinte étanche E à travers le sas d'entrée. En variante, les implants H peuvent être montés sur les tiges axiales verticales C de l'ascenseur L'. Il est également envisageable de monter plusieurs implants H sur une même tige axiale verticale C en utilisant une structure de support, tel que l'étrier Th.

Pour les étapes de lavage et de séchage ultérieures, il est prévu un casier de lavage W (figure 12e) sur lequel les implants greffés peuvent être déposés. Ce casier de lavage W comprend deux flasques d'extrémité W1 reliées entre elles par deux râteaux de rangement W2 et deux tiges de support W3. Les implants H peuvent ainsi être disposés entre les dents des râteaux W2 et en appui sur les tiges de support W3.

Il est à noter que certains aspects particuliers liés à la manipulation des implants fémoraux H peuvent être protégés indépendamment des autres caractéristiques déjà exposées et pourraient de ce fait faire l'objet de demandes divisionnaires. Plus particulièrement, l'étrier Th, la réglette V et le casier de lavage W pourraient faire l'objet de dépôts de brevet en soi.

Grâce à l'invention, on dispose d'un procédé de greffage de polymère bioactif sur des implants qui peut mis en œuvre de manière industrielle à très grande échelle. Les ustensiles qui ont été conçus, tels que l'élément de support S, la platine de support B, la plaque de support P, le casier de lavage D, l'étrier de support Th, la réglette V et le casier de lavage W permettent d'optimiser le procédé de greffage sur le plan industriel. Enfin, l'enceinte étanche E, et plus particulièrement son chariot et sa cuve rendent possibles une industrialisation à grande échelle de l'étape de polymérisation. Des implants, notamment dentaires et de hanche, peuvent ainsi être revêtus avec un polymère bioactif, tel que du PolyNaSS, à grande échelle et à grande cadence.

## Revendications

1. Procédé pour greffer du PolyNaSS sur des implants (I ; H) en titane ou alliage de titane, comprenant les étapes successives suivantes :
a) Monter des implants (I ; H) sur une structure de support d'implants (S ; Th),
b) Plonger les implants (I ; H) dans un bain d'acide pour les décaper,
c) Rincer les implants (I ; H),
d) Plonger les implants (I ; H) dans un bain d'anodisation pour les anodiser,
e) Rincer les implants (I ; H),
f) Introduire les implants (I ; H) dans une enceinte de polymérisation (E) remplie de gaz inerte, tel que l'argon,
g) Monter les implants sur un ascenseur (L ; L') présent dans l'enceinte de polymérisation (E),
h) Actionner l'ascenseur (L ; L') pour plonger les implants (I ; H) dans un bain de polymérisation présent dans l'enceinte de polymérisation (E),
i) Soumettre le bain de polymérisation à un catalyseur de polymérisation (M ; S_{UV}; S'_{UV}) pour synthétiser du polymère bioactif sur les implants (I ; H),
j) Remonter l'ascenseur (L ; L') pour extraire les implants du bain de polymérisation,
k) Retirer les implants (I ; H) de l'ascenseur (L ; L'),
l) Extraire les implants (I ; H) de l'enceinte de polymérisation (E),
m) Laver les implants (I ; H) pour les débarrasser de l'excès de polymère bioactif non greffé,
n) Sécher les implants greffés (I ; H).

2. Procédé selon la revendication 1, comprenant en outre une étape intermédiaire a1- entre l'étape a- et l'étape b-, cette étape a1-consistant à monter plusieurs implants (I) sur des éléments de support (S), eux-mêmes montés sur une platine de support (B), comprenant avantageusement un manche de préhension amovible (B4).

3. Procédé selon la revendication 2, dans lequel les implants (I), lors des étapes b- à f-, sont manipulés au moyen de la platine de support (B).

4. Procédé selon la revendication 1, 2 ou 3, comprenant en outre une étape intermédiaire e1- entre l'étape e- et l'étape f-, cette étape e1-consistant à placer la platine de support (B) avec ses éléments de support (S) d'implants (I) dans un récipient (R) rempli de gaz inerte, tel que l'argon, avantageusement pourvu d'un couvercle étanche (C), le récipient (R) étant ensuite introduit lors de l'étape f- dans l'enceinte de polymérisation (E) remplie de gaz inerte, tel que l'argon, le récipient (R) étant alors ouvert pour en extraire la platine de support (B) avec ses éléments de support (S) d'implants (I).

5. Procédé selon la revendication 4, comprenant en outre une étape intermédiaire f1- entre l'étape f- et l'étape g-, cette étape f1-consistant à retirer les éléments de support (S) d'implants de la platine de support (B), puis à monter les éléments de support (S) d'implants (I) sur une plaque de support (P) qui est ensuite montée sur l'ascenseur (L).

6. Procédé selon la revendication 4, comprenant en outre une étape intermédiaire f2- entre l'étape f- et l'étape g-, cette étape f2-consistant à monter sur des tiges axiales verticales (C) de ascenseur (L') qui sont entraînées en rotation et en révolution dans le bain de polymérisation, soit la platine de support (B), soit les éléments de support (S).

7. Procédé selon la revendication 5 ou 6, comprenant en outre une étape intermédiaire k1- entre l'étape k- et l'étape I-, cette étape k1-consistant à retirer les éléments de support (S) d'implants (I) de l'ascenseur (L ; L'), puis à monter les éléments de support (S) d'implants (I) sur une poutre centrale (D1) formant des logements de montage (D2) pour les éléments de support (S) d'implants (I) afin de former un panier le lavage (D), qui est ensuite extrait de l'enceinte de polymérisation (E).

8. Procédé selon la revendication 7, dans lequel, lors des étapes m- et n-, les éléments de support (S) d'implants (I) sont configurés sous la forme du panier de lavage (D).

9. Procédé selon la revendication 1, comprenant en outre une étape intermédiaire a2- entre l'étape a- et l'étape b-, cette étape a2-consistant à monter plusieurs implants (H) sur un étrier de support (Th), les implants (H), lors des étapes b- à f-, étant manipulés au moyen de l'étrier de support (B).

10. Procédé selon la revendication 9, comprenant en outre une étape intermédiaire f3- entre l'étape f- et l'étape g-, cette étape f3-consistant à monter les implants (H) côte à côte sur une réglette (V) qui est ensuite montée sur l'ascenseur (L).

11. Procédé selon la revendication 10, dans lequel, lors de l'étape I-, les implants (H) sont sur la réglette (V).

12. Procédé selon la revendication 9, 10 ou 11, dans lequel, lors des étapes m- et n-, les implants (H) sont disposés sur un casier de lavage (W).

## Patentansprüche

1. Verfahren zum Aufpfropfen von PolyNaSS auf Implantate (I; H) aus Titan oder einer Titanlegierung, umfassend die folgenden aufeinanderfolgenden Schritte:
a) Befestigen von Implantaten (I; H) auf einer Implantatträgerstruktur (S; Th),
b) Eintauchen der Implantate (I; H) in ein Säurebad, um sie zu ätzen,
c) Spülen der Implantate (I; H),
d) Eintauchen der Implantate (I; H) in ein Anodisierungsbad, um sie zu anodisieren,
e) Spülen der Implantate (I; H),
f) Einführen der Implantate (I; H) in eine mit Inertgas, zum Beispiel Argon, gefüllte Polymerisationskammer (E),
g) Anbringen der Implantate auf einem Aufzug (L; L'), der sich in der Polymerisationskammer (E) befindet,
h) Betätigen des Aufzugs (L; L'), um die Implantate (I; H) in ein Polymerisationsbad in der Polymerisationskammer (E) einzutauchen,
i) Aussetzen des Polymerisationsbads einem Polymerisationskatalysator (M; Suv; S'uv), um bioaktives Polymer auf den Implantaten (I; H) zu synthetisieren,
j) Hochfahren des Aufzugs (L; L'), um die Implantate aus dem Polymerisationsbad herauszuziehen,
k) Entfernen der Implantate (I; H) vom Aufzug (L; L'),
l) Herausziehen der Implantate (I; H) aus der Polymerisationskammer (E),
m) Waschen der Implantate (I; H), um überschüssiges, nicht transplantiertes bioaktives Polymer davon zu entfernen,
n) Trocknen der aufgepropften Implantate (1; H).

2. Verfahren nach Anspruch 1, ferner umfassend einen Zwischenschritt a1) zwischen Schritt a) und Schritt b), wobei dieser Schritt a1) darin besteht, mehrere Implantate (1) auf Trägerelementen (S) zu befestigen, die ihrerseits auf einer Trägerplatte (B) montiert sind, welche vorzugsweise mit einem abnehmbaren Haltegriff (B4) versehen ist.

3. Verfahren nach Anspruch 2, bei dem die Implantate (I) in den Schritten b) bis f) mit Hilfe der Trägerplatte (B) gehandhabt werden.

4. Verfahren nach Anspruch 1, 2 oder 3, ferner umfassend einen Zwischenschritt e1) zwischen Schritt e) und Schritt f), wobei Schritt e1) darin besteht, die Trägerplatte (B) mit ihren Implantatträgerelementen (S) in einen mit Inertgas, zum Beispiel Argon, gefüllten Behälter (R) einzusetzen, welcher vorteilhafterweise mit einem dichten Deckel (C) versehen ist, wobei der Behälter (R) dann in Schritt f) in die mit Inertgas, zum Beispiel Argon, gefüllte Polymerisationskammer (E) eingeführt wird, wobei der Behälter (R) anschließend geöffnet wird, um die Trägerplatte (B) mit ihren Implantatträgerelementen (S) herauszuziehen.

5. Verfahren nach Anspruch 4, ferner umfassend einen Zwischenschritt f1) zwischen Schritt f) und Schritt g), wobei Schritt f1) darin besteht, die Implantatträgerelemente (S) von der Trägerplatte (B) zu entfernen und anschließend die Implantatträgerelemente (S) auf einer Trägerplatte (P) zu befestigen, die dann auf dem Aufzug (L) angebracht wird.

6. Verfahren nach Anspruch 4, ferner umfassend einen Zwischenschritt f2) zwischen Schritt f) und Schritt g), wobei Schritt f2) darin besteht, an vertikalen axialen Stangen (C) des Aufzugs (L'), die drehend angetrieben und im Polymerisationsbad in Rotation versetzt werden, entweder die Trägerplatte (B) oder die Trägerelemente (S) anzubringen.

7. Verfahren nach Anspruch 5 oder 6, ferner umfassend einen Zwischenschritt k1) zwischen Schritt k) und Schritt I), wobei Schritt k1) darin besteht, die Implantatträgerelemente (S) vom Aufzug (L ; L') zu entfernen, dann die Implantatträgerelemente (S) an einem Mittelbalken (D1) zu befestigen, der Befestigungsaufnahmen (D2) für die Implantatträgerelemente (S) ausbildet, um einen Spülkorb (D) zu bilden, der dann aus der Polymerisationskammer (E) entnommen wird.

8. Verfahren nach Anspruch 7, bei dem in den Schritten m) und n) die Implantatträgerelemente (S) als Spülkorb (D) ausgebildet sind.

9. Verfahren nach Anspruch 1, ferner umfassend einen Zwischenschritt a2) zwischen Schritt a) und Schritt b), wobei Schritt a2) darin besteht, mehrere Implantate (H) auf einem Trägerbügel (Th) anzubringen, wobei die Implantate (H) in den Schritten b) bis f) mit Hilfe des Trägerbügels (B) gehandhabt werden.

10. Verfahren nach Anspruch 9, ferner umfassend einen Zwischenschritt f3) zwischen Schritt f) und Schritt g), wobei dieser Schritt f3) darin besteht, die Implantate (H) nebeneinander auf einer Leiste (V) anzuordnen, die dann am Aufzug (L) befestigt wird.

11. Verfahren nach Anspruch 10, bei dem in Schritt I) die Implantate (H) auf der Leiste (V) angeordnet sind.

12. Verfahren nach Anspruch 9, 10 oder 11, bei dem in den Schritten m) und n) die Implantate (H) auf einem Spülgestell (W) angeordnet sind.

## Claims

1. A method of grafting PolyNaSS, on implants (I; H) made of titanium or titanium alloy, the method comprising the following successive steps:
a) mounting implants (I; H) on an implant support structure (S; Th),
b) dipping the implants (I; H) into a bath of acid so as to clean them,
c) rinsing the implants (I; H),
d) dipping the implants (I; H) in an anodizing bath so as to anodize them,
e) rinsing the implants (I; H),
f) putting the implants (I; H) into a polymerization chamber (E) filled with inert gas, such as argon,
g) mounting the implants on an elevator (L; L') present in the polymerization chamber (E),
h) actuating the elevator (L; L') so as to dip the implants (I; H) into a polymerization bath present in the polymerization chamber (E),
i) subjecting the polymerization bath to a polymerization catalyst (M; S_{UV}; S'_{UV}) so as to synthesize bioactive polymer on the implants (I; H),
j) raising the elevator (L; L') so as to extract the implants from the polymerization bath,
k) removing the implants (I; H) from the elevator (L; L'),
l) extracting the implants (I; H) from the chamber (E),
m) washing the implants (I; H) so as to remove any excess non-grafted bioactive polymer therefrom,
n) drying the grafted implants (I; H).

2. A method according to claim 1, further comprising an intermediate step a1- between step a- and step b-, step a1- consisting in mounting a plurality of implants (I) on support elements (S) that are themselves mounted on a support slab (B) that advantageously includes a removable handle (B4).

3. A method according to claim 2, wherein, during steps b- to f-, the implants (I) are handled by means of the support slab (B).

4. A method according to claim 1, 2, or 3, further comprising an intermediate step e1- between step e- and step f-, step e1- consisting in placing the support slab (B) with its support elements (S) for supporting implants (I) in a container (R) filled with inert gas, such as argon, that is advantageously provided with a gastight lid (C), the container (R) then being put, during step f-, into the polymerization chamber (E) filled with inert gas, such as argon, the container (R) then being opened so as to extract the support slab (B) therefrom, together with its support elements (S) for supporting implants (I).

5. A method according to claim 4, further comprising an intermediate step f1- between step f- and step g-, step f1- consisting in removing the implant support elements (S) from the support slab (B), then in mounting the support elements (S) for supporting implants (I) on a support plate (P) that is then mounted on the elevator (L).

6. A method according to claim 4, further comprising an intermediate step f2- between step f- and step g-, step f2- consisting in mounting on vertical axial rods (C) of the elevator (L') that are rotated and revolve in the polymerization bath, either the support slab (B) or the support elements (S).

7. A method according to claim 5 or claim 6, further comprising an intermediate step k1- between step k- and step ℓ-, step k1- consisting in removing the support elements (S) for supporting implants (I) from the elevator (L; L'), then in mounting the support elements (S) for supporting implants (I) on a central pole (D1) that forms mounting housing (D2) for the support elements (S) for supporting implants (I) in order to form a washing rack (D) that is then extracted from the polymerization chamber (E).

8. A method according to claim 7, wherein, during steps m- and n-, the support elements (S) for supporting implants (I) are configured in the form of the washing rack (D).

9. A method according to claim 1, further comprising an intermediate step a2- between step a- and step b-, step a2- consisting in mounting a plurality of implants (H) on a support bracket (Th), the implants (H) being handled, during steps b- to f-, by means of the support bracket (B).

10. A method according to claim 9, further comprising an intermediate step f3- between step f- and step g-, step f3- consisting in mounting the implants (H) side-by-side on a strip (V) that is then mounted on the elevator (L).

11. A method according to claim 10, wherein, during step ℓ-, the implants (H) are on the strip (V).

12. A method according to claim 9, 10, or 11, wherein, during steps m- and n-, the implants (H) are arranged on a washing tray (W).
